# EUROPEAN PATENT APPLICATION

(11) **EP 0 696 453 A2**
(43) Date of publication of application: **14.02.1996**
(21) Application number: 95201579.0
(22) Date of filing: 14.06.1995
(51) Int. Cl.: A61K 31/20, A23L 1/30, A23D 7/00, A61K 9/10

(54) **Pediatric lipid emulsion**

(30) Priority: 17.06.1994 US 261413
(71) Applicant: CLINTEC NUTRITION COMPANY, Deerfield, Illinois 60015-0760 (US)
(72) Inventor: Trimbo, Susan L., Evanston, IL 60202 (US)
(74) Representative: Vuille, Roman

(57) **Abstract**

The present invention provides an improved pediatric lipid emulsion. The lipid emulsion can be constructed so that it is administered either enterally or parenterally. Pursuant to the present invention, the pediatric lipid emulsion comprises a source of linoleic acid, a source of alpha-linolenic acid, a source of gamma linolenic acid, a source of docosahexaenoic acid (DHA), a source of medium chain triglycerides, and a source of oleic acid. Methods for providing lipids to a neonate are also provided.

## Description

The present invention relates to compositions and therapies for treating patients. More specifically, the present invention relates to compositions for meeting nutritional requirements in neonatal and pediatric patients.

It is known to provide nutritional requirements to a patient through the use of enteral and parenteral formulations. Enteral formulations include those formulations that are administered to the patient through the patient's naso-gastric system. These formulations can either be administered as a drink to the patient or infused into the patient through a tube, such as a naso-gastric enteral feeding tube.

It is also known to provide nutritional requirements to a patient through the use of a parenteral route. An example of a parenteral route is through intravenous infusion.

These nutritional formulations can either provide specific nutrients and components, and therefore be used as an adjunct to other nutritional sources, or they can provide the complete nutritional requirements of the patient. Likewise, parenteral nutritional support can supplement oral intake. Accordingly, a number of enteral and parenteral formulations are available.

One type of nutritional formulation is a lipid emulsion which provides fatty acids and energy to the patient. Lipid emulsions can either be parenteral or enteral formulations.

There are situations wherein pediatric patients require lipid emulsions. However, it is the current belief of the inventor of the present invention, that currently available pediatric lipid emulsions do not meet neonatal fatty acid requirements. For example, based on information and belief, Kabi Vitrum intends to market a pediatric lipid emulsion that is comprised of soy oil and borage oil in proportions of 93:7 w/w. The formulation primarily provides: linoleic acid (18:2 n-6); gamma-linolenic acid (18:3 n-6); and alpha-linolenic (18:3 n-3). However, such a formulation does not meet the total fatty acid requirements of a neonate. An inadequate or unbalanced mixture of fatty acids can have many detrimental effects on a neonate. The failure to provide the necessary fatty acids to the neonate can lead to impaired growth in the neonate. Furthermore, a failure to provide the proper balance and intake of fatty acids can adversely affect neural development in a neonate.

Some of the limitations of existing lipid emulsions as discussed in: O. Goulet, *Long Term Utilization of a γ-Linolenic Acid Enriched Intravenous Fat Emulsion in Children,* Clin. Nutr. 12:(Supp 2), 1993; Martinez et al, *Effects of Parenteral Nutrition with High Doses of Linoleate on the Developing Human Liver and Brain,* Lipids, Vol. 22, No. 3 (1987); Carlson et al, *Visual-Acuity Development in Healthy Preterm Infants: Effect of Marine-Oil Supplementation*, Am. J. Clin. Nutr., 1993:58:35-42; Neuringer et al, *The Essentiality of N-3 Fatty Acids for the Development and Function of the Retina and Brain,* Ann. Rev. Nutr., 1988, 8:517-41; Clandinin et al, *Requirements of Newborn Infants for Long Chain Polyunsaturated Fatty Acids*, Acta Poediatr. Scand. Suppl., 351:63-71, 1989; Hoffmann et al, *Effects of* *Supplementation with ω3 Long-Chain Polyunsaturated Fatty Acids on Retinal and Cortical Development in Premature Infants*, Am. J. Clin. Nutr., 1993:57(suppl):807S-12S; N. Salem Jr. et al, *Docosahexaenoic Acid is an Essential Nutrient in the Nervous System*, J. Nutr. Sci. Vitaminol, 1992, 153-6; Koletzko et al*, Effects of Dietary Long-Chain Polyunsaturated Fatty Acids on the Essential Fatty Acid Status of Premature Infants*, European Journal of Pediatrics, (1989) 148:669-675; Clark et al, *Determination of the Optimal Ratio of Linoleic Acid to α-Linolenic Acid in Infant Formulas*, The Journal of Pediatrics, April 1992, S151-S158; Innis et al, *n-3 Fatty Acid Requirements of the Newborn*, Lipids, Vol. 27, No. 11 (1992); Hoffmann et al, *Essentiality of Dietary ω3 Fatty Acids for Premature Infants: Plasma and Red Blood Cell Fatty Acid Composition*, Lipids, Vol. 27, No. 11 (1992); Aggett et al, *Comment on the Content and Composition of Lipids in Infant Formulas*, Acta. Poediatr. Scand., 80: 887-896, 1991; and European Patent Application No. 0 400 547.

Accordingly, there is a need for an improved lipid emulsion for pediatric use.

The present invention provides an improved pediatric lipid emulsion. The lipid emulsion can be constructed so that it is administered either enterally or parenterally. Pursuant to the present invention, the pediatric lipid emulsion comprises a source of linoleic acid, a source of linolenic acid (ALA, 18:3 n-3 and GLA, 18:3 n-6), a source of docosahexaenoic acid (DHA), a source of medium chain triglycerides, and a source of oleic acid.

In an embodiment, the DHA comprises less than 5.0% of the fatty acids, by caloric content, provided by the emulsion.

In an embodiment, the ratio of n-6 to n-3 fatty acids is less than or equal to 10:1.

In an embodiment, the ratio of n-6 to n-3 fatty acids present in the emulsion is between 1:1 to about 5:1; the preferred ratio being approximately 4:1-5:1.

In an embodiment, MCTs comprise less than or equal to 50%, by caloric content, of the fatty acids present in the emulsion.

In an embodiment, the source of gamma-linolenic acid (GLA) is chosen from borage oil, black currant seed oil, and evening primrose oil. In a further embodiment, the gamma-linolenic acid comprises less than or equal to 15%, by caloric content, of the fatty acids.

In an embodiment, the linoleic acid source is chosen from the group consisting of: sunflower; safflower; soybean; corn; and canola oils.

In an embodiment, the alpha-linolenic (18:3 n-3) source is chosen from the group consisting of: soybean oil; and canola oil.

In an embodiment, the gamma-linolenic acid (18:3 n-3) source is chosen from the group consisting of: black currant seed; borage; and evening primrose oil.

In an embodiment, the oleic acid is chosen from the group consisting of: canola; olive; and the high oleic acid variants of sunflower and safflower oils.

In a further embodiment, a pediatric lipid emulsion is provided comprising: approximately 15 to about 35% MCTs, by calories, of the total fatty acids; approximately 40 to about 65% of the total fatty acids, by calories, being provided by at least one oil chosen from the group consisting of: canola, soy, and olive oil; approximately 10 to about 25% of at least one oil chosen from the group consisting of borage and black currant oil; and 0 to about 20% marine oil.

The present invention also provides a method for providing fatty acids to a pediatric patient. The method comprises the step of administering to the patient a therapeutically effective amount of a lipid emulsion comprising: a source of linoleic acid; a source of linolenic (ALA and GLA) acid; a source of DHA; a source of medium chain triglycerides; and a source of oleic acid.

In an embodiment, the lipid emulsion is administered enterally.

In an embodiment, the lipid emulsion is administered parenterally.

It is an advantage of the present invention to provide, in an emulsion, the necessary fatty acids required by a neonate.

Still further, an advantage of the present invention is to provide a method for meeting the fatty acid requirements of an infant.

Furthermore, an advantage of the present invention is that it provides an enteral lipid emulsion for pediatric patients.

Moreover, an advantage of the present invention is that it provides a parenteral lipid emulsion for pediatric patients.

Additionally, an advantage of the present invention is that the lipid emulsions of the present invention more closely approximate the fatty acid requirements of neonates compared to other commercial products.

Additional features and advantages of the present invention are described in, and will be apparent from, the detailed description of the presently preferred embodiments.

The present invention provides a pediatric lipid emulsion. The emulsion has an optimum mixture of the lipids that are necessary for premature and term infants and pediatric patients. The formulation includes a mixture of oils that provides a preferred amount and balance of fatty acids. The formulation can be constructed so that it can be administered either enterally or parenterally.

It has been determined that commercially available enteral and parenteral lipid emulsion products provide an inadequate amount and/or mixture of fatty acids for neonates or pediatric patients. This inadequate fatty acid content can result in the neonate failing to receive the proper intake of fatty acids, leading to adverse and detrimental results.

It has also been determined that for optimum development, infants require the following fatty acids: linoleic acid; linolenic acid; DHA; and possibly oleic acid. Medium chain triglycerides are not required but serve as an excellent fuel source and are well absorbed from enteral diets. By providing, in an emulsion, the proper balance of these fatty acids, the fatty acid requirements of a neonate can be met.

The linoleic acid can be provided by a number of different oils. Preferably, linoleic acid is provided by: sunflower oil; canola oil; soy oil; and/or safflower oil and mixtures of same. Preferably, the source of linoleic acid comprises approximately 2 to about 10% of the total calories of the composition.

Alpha-linolenic acid (ALA) can also be provided by a number of oils. Preferably, the linolenic acid is provided by: soybean oil; canola oil; and mixtures of same. Preferably, the source of alpha-linolenic provides less than or equal to 3% of the total calories. The linolenic to alpha-linoleic acid ratios should be less than 10:1.

Gamma-Linoleic acid (GLA) can be provided by black currant seed oil, borage oil, and evening primrose oil. GLA may be included in amounts of less than 2% of the calories.

Preferably, the composition provides DHA through the use of marine oil. Preferably, the DHA intake is less than 5.0%, by caloric content, of the fatty acids. In an embodiment, 0 to about 20% of the total calories of the composition are provided as marine oil. As used herein, "marine oil" not only refers to fish oil, but to oils that are derived from other aquatic organisms, such as, algae. Purified fatty acid esters may also be used as a source of DHA. Additionally, in certain situations, it may be important to maintain a specific ratio of n-6 to n-3 fatty acids. In a preferred embodiment, the ratio is 1:1 to about 5:1.

Alpha-linolenic acid may be used as the sole source of n-3 fatty acids for pediatric patients since they are better able to synthesize DHA from alpha-linolenic.

It may be desirable to provide gamma-linolenic acid in the composition particularly for the neonate. The source of gamma-linolenic acid should not exceed 15% of the total caloric content of the fatty acids.

As previously stated, the composition includes a source of oleic acid. Oleic acid can be provided by a number of different oils. Preferably, high oleic acid variants of sunflower and safflower oils, olive, and/or canola oil and blends of same are used as a source of oleic acid.

Preferably, the source of oleic acid comprises up to 60% of the total calories of the composition.

The phospholipids that are used to emulsify the oils can also serve as a source of any or all of the critical fatty acids.

Additionally, the composition includes medium chain triglycerides. The medium chain triglycerides preferably represent no more than 50%, by caloric content, of the oils/fatty acids.

The ideal proportions of various fatty acids needed by neonates and pediatric patients may change with maturation. For example, neonates may require DHA; this fatty acid is less important to pediatric patients.

A variety of lipid emulsions can be created pursuant to the present invention.

For example, the fatty acid source of the lipid emulsion can comprise: approximately 20% MCTs; approximately 45 to about 50% canola oil; approximately 15% borage oil; and approximately 10 to about 15% marine oil. If desired, canola/soy or olive oil/soy blend can be substituted for the canola oil. Likewise, if desired, black currant oil can be substituted for the borage oil.

Another option is a fatty acid source that comprises: approximately 20 to about 30% MCTs; approximately 50 to about 60% canola oil; and approximately 20% borage oil. If desired, canola/soy or olive oil/soy blend can be substituted for the canola oil.

Preferably, in addition to the lipids, the emulsion includes: egg yolk phosphatide as an emulsifier, glycerol for isotonicity, sterile water for injection as a diluent, and sodium hydroxide for pH adjustment.

By way of example, and not limitation, examples of the present invention are as follows:

### EXAMPLE NO. 1

### 20% Intravenous Pediatric Lipid Emulsion Composition

| **Per 100 ml** | **Ingredient** |
|---|---|
| 20 g | Oils |
| | MCT oil (15%) |
| | Canola oil (60%) |
| | Menhaden oil (15%) |
| | Black currant seed oil (10%) |
| 2.25 g | Glycerol, USP |
| 1.2 g | Egg yolk phosphatide |
| q.s. | Water for injection, USP |

In a suitable vessel, the oils, glycerol, water, and egg phosphatide are mixed to produce an emulsion. The emulsion is homogenized repeatedly under pressure to produce a mean particle size of 0.75 µm. The pH is adjusted in the process to approximately 7.0 with sodium hydroxide. The final volume is adjusted with water for injection to the desired concentration and the emulsion is put into glass containers and heat sterilized.

### EXAMPLE NO. 2

### Composition of Pediatric Enteral Diet

| **Component** | **% Calories** | **Source** |
|---|---|---|
| Protein | 12 | Hydrolyzed whey protein |
| Carbohydrate | 48 | Maltodextrin, cornstarch, sucrose |
| Lipid | 40 | Canola oil (60%), medium-chain triglyceride oil (20%), borage oil (8%), marine oil (10%) |
| Emulsifier | | Egg yolk phosphatide (2%) |
| Vitamins and Minerals | | In accordance with NAS-NRC* recommendations for age group |

| | | |
|---|---|---|
| *National Academy of Sciences-National Research Council | | |

### EXAMPLE NO. 3

### Composition of a Neonatal Enteral Diet

| **Component** | **g/100 kcal** | **Source** |
|---|---|---|
| Protein | 3 | Casein-whey mixture |
| Carbohydrate | 5 | Maltodextrin, cornstarch, lactose |
| Lipid | 11 | Soybean oil (45%), olive oil (25%), black currant seed oil (8%), marine oil (5%), medium-chain triglycerides (15%), egg phosphatide (2%) |

| **Component** | **Per 100 kcal** | |
|---|---|---|
| Vitamins & Minerals | | |
| Calcium (mg) | 110 | |
| Phosphorus (mg) | 60 | |
| Magnesium (mg) | 5 | |
| Iron (mg) | 0.3 | |
| Zinc (mg) | 1.5 | |
| Manganese (mg) | 15 | |
| Copper (mcg) | 160 | |
| Iodine (mcg) | 10 | |
| Sodium (mg) | 40 | |
| Potassium (mg) | 110 | |
| Chloride (mg) | 90 | |
| Vitamin A (IU) | 800 | |
| VitaminD (IU) | 150 | |
| Vitamin E (IU) | 4 | |
| Vitamin K (mcg) | 10 | |
| Thiamin (mcg) | 200 | |
| Riboflavin (mcg) | 300 | |
| Vitamin B₆ (mcg) | 200 | |
| Vitamin B₁₂ (mcg) | 0.5 | |
| Niacin (mcg) | 3000 | |
| Folic acid (mcg) | 30 | |
| Pantothenic acid (mcg) | 1000 | |
| Vitamin C (mg) | 25 | |

By way of example, and not limitation, contemplative examples of the present invention will now be given

### Contemplative Example 1A

### Method of Use

The following is a contemplative example of the use of an intravenous, 20% pediatric lipid emulsion.

An infant weighing 2500 g requiring TPN was administered the following regimen: 2.5 g amino acids/kg body weight, 2.5 g lipid/kg body weight, 20 g glucose/kg body weight. TPN would be administered for 7 days beginning on Day 3 after birth (16 hours per day) at which time the infant will be transitioned to enteral feeding. The parenteral feeding should be well tolerated as will be evidenced by what Applicant anticipates will be the resultant parameters:

| **Clinical/Biochemical Parameters** | **Day 3** | **Day 10** |
|---|---|---|
| Body Weight (g) | 2500 | 2550 |
| Bilirubin (mg%) | 6.0 | 3.5 |
| Serum Triglycerides (mg/dl) | 50 | 75 |

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. A pediatric lipid emulsion comprising:
a source of linoleic acid;
a source of alpha-linolenic acid;
a source of gamma-linolenic acid;
a source of docosahexaenoic acid;
a source of medium chain triglycerides; and
a source of oleic acid.

2. A pediatric lipid emulsion according claim 1 in which the linoleic acid source is selected from soybean oil, safflower oil, canola oil, corn oil, and sunflower oil, or mixtures thereof.

3. A pediatric lipid emulsion according to claim 1 or claim 2 in which the gamma-linoleic acid source is selected from borage oil, black currant oil, and evening primrose oil.

4. A pediatric lipid emulsion according to any one of claims 1 to 3 in which the alpha-linolenic acid source is selected from soybean oil and canola oil, or mixtures thereof.

5. A pediatric lipid emulsion comprising:
about 15 to about 35%, on the basis of the total calories of the total fatty acids, of medium chain triglycerides;
about 40 to about 65%, on the basis of the total calories of the total fatty acids, of at least one oil selected from canola oil, soybean oil, and olive oil to provide a source of linoleic acid, alpha-linolenic acid, and oleic acid;
about 10 to about 25% of the total calories of the fatty acids of at least one oil chosen from borage oil and black currant oil to provide a source of gamma-linolenic acid; and
about 0 to about 20% marine oil to provide a source of docosahexaenoic acid.

6. A pediatric lipid emulsion according to any one of claims 1 to 5 in which the docosahexaenoic acid comprises less than 5.0% of total fatty acids, on the basis of caloric content, provided by the emulsion.

7. A pediatric lipid emulsion according to any one of claims 1 to 6 in which the ratio of linoleic acid to alpha-linolenic acids is less than 10:1.

8. A pediatric lipid emulsion according to any one of claims 1 to 7 in which the ratio of n-6 to n-3 fatty acids present in the emulsion is between about 1:1 to about 5:1.

9. A pediatric lipid emulsion according to any one of claims 1 to 8 in which the medium chain triglycerides comprise less than or equal to 50% of total fatty acids, on the basis of caloric content, provided by the emulsion..

10. A pediatric lipid emulsion according to any one of claims 1 to 9 in which the gamma-linolenic acid comprises less than or equal to 15% of total fatty acids, on the basis of caloric content, provided by the emulsion.
